# EUROPEAN PATENT APPLICATION

(11) **EP 4 755 329 A1**
(43) Date of publication of application: **10.06.2026**
(21) Application number: 25216319.1
(22) Date of filing: 17.11.2025
(51) Int. Cl.: A61B 18/14, A61B 5/00

(54) **APPARATUSES FOR CATHETER-BASED ELECTROPHYSIOLOGY**

(30) Priority: 05.12.2024 US 202418969596
(71) Applicant: Biosense Webster (Israel) Ltd., 2066717 Yokneam (IL)
(72) Inventor: KEYES, Joseph Thomas, Irvine, 92618 (US); BEECKLER, Christopher Thomas, Irvine, 92618 (US); ALEXANDER, Larissa, Irvine, 92618 (US)
(74) Representative: Carpmaels & Ransford LLP

(57) **Abstract**

The present disclosure provides a flexible circuit strip for use in a catheter assembly. The flexible circuit strip comprises a first insulating layer, a second insulating layer and a third insulating layer, each having an elongated shape and bonded together such that the second insulating layer is between the first and third insulating layers; a conductive trace layer positioned between the first and second insulating layers, the conductive trace layer including longitudinal circuit traces for carrying electrical signals; electrodes positioned along a length of an outer surface of the first insulating layer and electrically connected to the circuit traces; and a longitudinal hollow channel formed between any two of the first, second and third layers, the longitudinal hollow channel being configured to receive an elongated support element.

## Description

### FIELD

The presently disclosed subject matter generally relates to the field of medical devices and in particular to catheters for electrophysiology.

### BACKGROUND

Cardiac arrhythmias, such as atrial fibrillation (AF), occur when regions of cardiac tissue abnormally conduct electric signals to adjacent tissue. This disrupts the normal cardiac cycle and causes asynchronous rhythm. Certain procedures exist for treating arrhythmia, including surgically disrupting the origin of the signals causing the arrhythmia and disrupting the conducting pathway for such signals. By selectively ablating cardiac tissue by application of energy via a catheter, it is sometimes possible to cease or modify the propagation of unwanted electrical signals from one portion of the heart to another.

Many current ablation approaches in the art tend to utilize radiofrequency (RF) electrical energy to heat tissue. RF ablation can have certain rare drawbacks due to operator's skill, such as heightened risk of thermal cell injury which can lead to tissue charring, burning, steam pop, phrenic nerve palsy, pulmonary vein stenosis, and esophageal fistula. Cryoablation is an alternative approach to RF ablation that can reduce some thermal risks associated with RF ablation but may present tissue damage due to the very low temperature nature of such devices. Maneuvering cryoablation devices and selectively applying cryoablation, however, is generally more challenging compared to RF ablation; therefore, cryoablation is not viable in certain anatomical geometries which may be reached by electrical ablation devices.

Some ablation approaches use irreversible electroporation (IRE) to ablate cardiac tissue using nonthermal ablation methods. IRE delivers short pulses of high voltage to tissues and generates an unrecoverable permeabilization of cell membranes. Delivery of IRE energy to tissues using multi-electrode catheters was previously proposed in the patent literature. Examples of systems and devices configured for IRE ablation are disclosed in U.S. Patent Pub. No. 2021/0169550A1, 2021/0169567A1, 2021/0169568A1, 2021/0161592A1, 2021/0196372A1, 2021/0177503A1, and 2021/0186604A1.

Regions of cardiac tissue can be mapped by a catheter to identify the abnormal electrical signals. The same or different catheter can be used to perform ablation. Some example catheters include a number of spines with electrodes positioned thereon. The electrodes are generally attached to the spines and secured in place by soldering, welding, or using an adhesive. Furthermore, multiple linear spines are generally assembled together by attaching both ends of the linear spines to a tubular shaft (e.g., a pusher tube) to form a spherical basket. Due to the small size of the spines and the electrodes, however, adhering the electrodes to the spines and then forming a spherical basket from the multiple linear spines can be a difficult task, increasing the manufacturing time and cost and the chances that the electrode fails due to an improper bond or misalignment. What is needed, therefore, are devices and methods of forming an improved basket assembly that can help to reduce the time required for manufacturing the basket assembly, alternative catheter geometries, and alternative electrode shapes and sizes in general.

U.S. Patent Application Publication No. 2010/0305420 A1 provides a flexible circuit that includes conductive material on the top and bottom planar surfaces of a dielectric substrate. The flexible circuit can be used in various applications, including use as a sensor. A via is used to provide electrical communication between the top and bottom surface of the flexible circuit. A method of preparing a flexible circuit and a medical instrument including the flexible circuit are also provided.

U.S. Patent No. 9,171,794 B2 provides systems and methods for the embedding of thin chips. A well region is generated in a substrate that includes a conductive material disposed on a flexible polymer. The standoff well region can be generated by pattern the conductive material, where the thin chip is embedded in the standoff well region. A cavity can be generated in the polymer layer to form a polymer well region, where the thin chip is embedded in the polymer well region.

U.S. Patent No. 10,362,953 B2 provides a catheter with an electrode array formed by an interconnected framework. The framework may have a plurality of elements interconnected by a plurality of junctions at locations intermediate the proximal and distal ends of the electrode array assembly. The electrodes may be printed on a polymeric layer of the interconnected framework.

U.S. Patent No. 10,297,572 B2 provides flexible interconnects, flexible integrated circuit systems and devices, and methods of making and using flexible integrated circuitry. A flexible integrated circuit system is disclosed which includes first and second discrete devices that are electrically connected by a discrete flexible interconnect. The first discrete devices include a first flexible multi-layer integrated circuit (IC) package with a first electrical connection pad on an outer surface thereof. The second discrete device includes a second flexible multi-layer integrated circuit (IC) package with a second electrical connection pad on an outer surface thereof. The discrete flexible interconnect is attached to and electrically connects the first electrical connection pad of the first discrete device to the second electrical connection pad of the second discrete device.

U.S. Patent No. 10,681,805 B2 provides an improved flexible circuit structure, for example, by providing fault-tolerant electrical pathways for flow of electric current through the flexible circuit structure. In some embodiments, such fault tolerance is enhanced by way of a conductive mesh provided between an adjacent pair of resistive elements. Some aspects are related to improved voltage, current, or voltage and current measurement associated with various pairs of adjacent resistive elements at least when the various pairs have differing distances between them.

U.S. Patent No. 9,159,635 B2 provides a flexible electronic structure and methods for fabricating flexible electronic structures. An example method includes applying a first layer to a substrate, creating a plurality of vias through the first layer to the substrate, and applying a second polymer layer to the first layer such that the second polymer forms anchors contacting at least a portion of the substrate. At least one electronic device layer is disposed on a portion of the second polymer layer. At least one trench is formed through the second polymer layer to expose at least a portion of the first layer. At least a portion of the first layer is removed by exposing the structure to a selective etchant to providing a flexible electronic structure that is in contact with the substrate. The electronic structure can be released from the substrate.

### SUMMARY

The present disclosure addresses the technical challenges associated with manufacturing catheters for use in catheter-based electrophysiology, particularly for mapping and ablation procedures. Electrophysiology catheters typically include an expandable end effector including flexible splines forming - in its an expanded form - a basket shape. Current methods for manufacturing such flexible splines include laminating flexible circuit strips onto elongated flexible support members, such as nitinol and use of heat shrink sleeves. These processes become increasingly complex and costly when dealing with shape-set nitinol or other materials with intricate three-dimensional geometries. In particular, traditional methods are prone to issues like improper bonding, misalignment, and the need for costly and labor-intensive steps such as laser ablation to create openings for exposing the electrodes subsequently to the use of heat shrink sleeves.

The presently disclosed subject matter proposes creating a flexible circuit strip that incorporates a longitudinal hollow channel during the fabrication process. This channel is designed to accommodate the elongated support element, such as shape-set nitinol, without the need for numerous additional assembly steps post-fabrication. By integrating the channel into the flexible circuit strip, the assembly process is simplified, reducing time and cost while improving the reliability and performance of the final product.

The longitudinal hollow channel may in particular be formed by etching away a conductive layer (e.g., copper) while preserving other conductive elements such as gold traces and electrodes. This channel is designed to receive an elongated support element, allowing for ease of assembly. The flexible circuit strip with its integrated channel can accommodate shape-set nitinol and other materials with various geometries.

### BRIEF DESCRIPTION OF THE DRAWINGS

In order to better understand the subject matter that is disclosed herein and to exemplify how it may be carried out in practice, examples will now be described, by way of non-limiting example only, with reference to the accompanying drawings, in which:
**FIG. 1** illustrates a catheter-based electrophysiology mapping and ablation system, according to examples of the presently disclosed subject matter;
**FIG. 2** illustrates a catheter assembly according to examples of the present disclosure;
**FIG. 3** illustrates a top view of flexible circuit strips according to examples of the present disclosure;
**FIG. 4** illustrates a cross section view of a flexible circuit strip according to examples of the present disclosure;
**FIGS. 5A-5C** illustrate views of a flexibles circuit strip according to other examples of the present disclosure;
**FIG. 6** illustrates steps of a method for fabricating a flexible circuit strip according to examples of the present disclosure.

### DETAILED DESCRIPTION

As used herein, the terms "about" or "approximately" for any numerical values or ranges indicate a suitable dimensional tolerance that allows the part or collection of components to function for its intended purpose as described herein. More specifically, "about" or "approximately" can refer to the range of values ±20% of the recited value, e.g. "about 90%" can refer to the range of values from 71% to 99%.

As discussed herein, vasculature of a "subject" or "patient" can be vasculature of a human or any animal. It should be appreciated that an animal can be a variety of any applicable type, including, but not limited thereto, mammal, veterinarian animal, livestock animal or pet type animal, etc. As an example, the animal can be a laboratory animal specifically selected to have certain characteristics similar to a human (e.g., rat, dog, pig, monkey, or the like). It should be appreciated that the subject can be any applicable human patient, for example.

As discussed herein, "operator" can include a doctor, physician, surgeon, or any other individual or delivery instrumentation associated with delivery of a multi-electrode RF balloon catheter for the treatment of drug refractory atrial fibrillation to a subject and the term "proximal" means that an object is closer to the "operator" and "distal" means that the object is further away from the "operator".

As used herein in the context of a circuit strip, the term 'longitudinal' refers to the direction along the length of the strip, extending from the proximal end to the distal end. The term 'lateral' refers to the direction perpendicular to the longitudinal axis, spanning the width of the strip. The term 'thickness' refers to the dimension perpendicular to both the longitudinal and lateral directions, indicating the depth of the strip from the top surface (i.e. top layer) to the bottom surface (bottom layer).

In the following detailed description, numerous specific details are set forth in order to provide a thorough understanding. However, it will be understood by those skilled in the art that the presently disclosed subject matter may be practiced without these specific details. In other instances, well-known methods and features have not been described in detail so as not to obscure the presently disclosed subject matter.

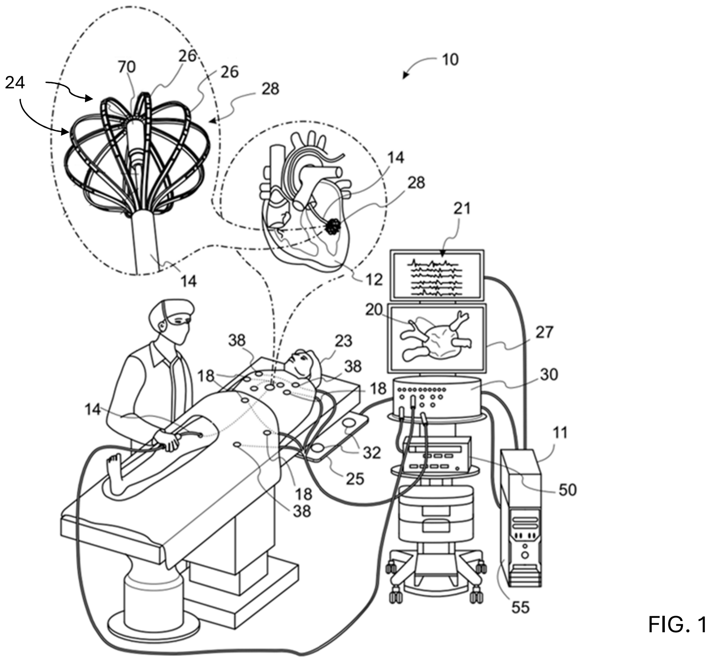

Reference is made to **FIG. 1** showing an example catheter-based electrophysiology mapping and ablation system **10.** System **10** includes multiple catheters, which are percutaneously inserted by a physician through the patient's vascular system into a chamber or vascular structure of a heart **12.** Typically, a delivery sheath catheter is inserted into the left or right atrium near a desired location in heart **12.** Thereafter, one or more catheters may be inserted into the delivery sheath catheter so as to arrive at the desired location in heart **12.** The plurality of catheters may include catheters dedicated for sensing Intracardiac Electrogram (IEGM) signals, catheters dedicated for ablating and/or catheters dedicated for both sensing and ablating. An example catheter **14** that is configured for ablating tissue and/or for sensing electrical cardiac activity and/or mapping is illustrated herein.

Catheter **14** is an exemplary catheter having an end effector at its distal tip **28** that includes an expandable assembly, having one and preferably multiple electrodes **26** optionally distributed over a plurality of flexible spline elements **24.** The electrodes **26** are generally configured for delivering ablation energy to tissue and/or for sensing cardiac electrical signals. Catheter **14** additionally includes one or more position sensors **70** embedded in or near distal tip **28** for tracking position and orientation of distal tip **28.** Optionally and preferably, position sensor **70** is a magnetic based position sensor, for example a position sensor including three magnetic coils for sensing three-dimensional (3D) position and orientation; or a position sensor including one magnetic coil, for sensing a single direction.

Each of the magnetic based position sensors **70** may be operated together with a location pad **25** including a plurality of magnetic coils **32** configured to generate magnetic fields in a predefined working volume. Real time position of distal tip **28** of catheter **14** may be tracked based on magnetic fields generated with location pad **25** and sensed by magnetic based position sensor **70.** Details of the magnetic based position sensing technology are described in U.S. Patent Nos. 5,391,199; 5,443,489; 5,558,091; 6,172,499; 6,239,724; 6,332,089; 6,484,118; 6,618,612; 6,690,963; 6,788,967; 6,892,091.

The physician may place a distal tip **28** of catheter **14** in contact with the heart wall for sensing a target site in heart **12.** For ablation, the physician may similarly place a distal end of an ablation catheter in contact with a target site for ablating tissue.

System 10 includes one or more electrode patches **38** positioned for skin contact on patient **23** to establish location reference for location pad **25** as well as impedance-based tracking of electrodes **26.** For impedance-based tracking, electrical current is directed to electrodes **26** and sensed at electrode skin patches **38** so that the location of each electrode can be triangulated via the electrode patches **38.** Details of the impedance-based location tracking technology are described in US Patent Nos. 7,536,218; 7,756,576; 7,848,787; 7,869,865; and 8,456,182.

A recorder **11** records and displays electrograms **21** captured with body surface ECG electrodes **18** and intracardiac electrograms (IEGM) captured with electrodes **26** of catheter **14.** Recorder **11** may include pacing capability for pacing the heart rhythm and/or may be electrically connected to a standalone pacer.

System **10** may include an ablation energy generator **50** that is adapted to conduct ablative energy to one or more of electrodes at a distal tip of a catheter configured for ablating. Energy produced by ablation energy generator **50** may include, but is not limited to, radiofrequency (RF) energy or pulsed-field ablation (PFA) energy, including monopolar or bipolar high-voltage DC pulses as may be used to effect irreversible electroporation (IRE), or combinations thereof.

Patient interface unit (PIU) **30** is an interface configured to establish electrical communication between catheters, other electrophysiological equipment, power supply and a workstation **55** for controlling operation of system **10.** Electrophysiological equipment of system **10** may include for example, multiple catheters, location pad **25,** body surface ECG electrodes **18,** electrode patches **38,** ablation energy generator **50,** and recorder **11.** Optionally and preferably, PIU **30** additionally includes processing capability for implementing real-time computations of location of the catheters and for performing ECG calculations.

Workstation **55** includes memory, processor unit with memory or storage with appropriate operating software stored therein, and user interface capability. Workstation **55** may provide multiple functions, optionally including (1) modeling the endocardial anatomy in three-dimensions (3D) and rendering the model or anatomical map **20** for display on a display device **27,** (2) displaying on display device **27** activation sequences (or other data) compiled from recorded electrograms **21** in representative visual indicia or imagery superimposed on the rendered anatomical map **20,** (3) displaying real-time location and orientation of multiple catheters within the heart chamber, and (4) displaying on display device **27** sites of interest such as places where ablation energy has been applied. One commercial product embodying elements of the system **10** is available as the CARTO^{™} 3 System, available from Biosense Webster, Inc., 31A Technology Drive, Irvine, CA 92618.

In some examples, an irrigation module is provided for delivering irrigation fluid, such as saline solution, to the location of treatment. The irrigation module may comprise a pump and an associated fluid tank.

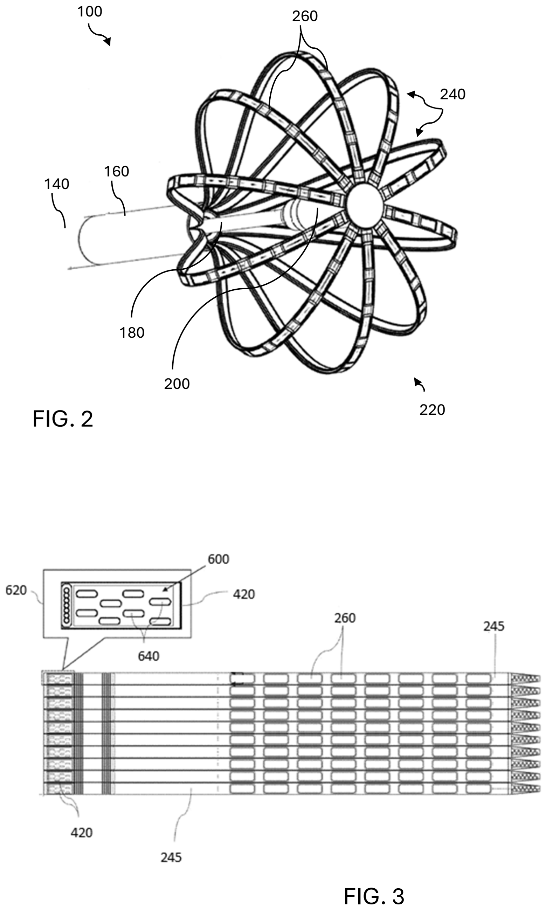

Reference is now made to **FIG. 2****,** which is schematic view of a catheter assembly **100** constructed and operative in accordance with examples of the present disclosure. The catheter assembly **100** include a tubular shaft **140** having a distal end, a coupler (also referred to as spline retention hub) **160** connected to the distal end **140** of the tubular shaft and a pusher **180** including a distal portion **200.** The pusher **180** may be configured to be advanced and retracted through the tubular shaft, for example, using a manipulator or handle (not shown). The pusher **180** may be disposed coaxially within the spline retention hub and configured to be translated longitudinally relative thereto. The catheter assembly **100** also includes an expandable assembly **220** (also referred to generally as expandable end effector) comprising a plurality of flexible splines **240** (only one label for the sake of simplicity). Each flexible spline **240** includes a flexible circuit strip (PCB) including multiple electrodes **260** disposed thereon (only some labeled for the sake of simplicity) and a corresponding elongated resilient support element extending along a given length of the flexible polymer circuit strip. The flexible splines **240** may be coupled at one end to the coupler **160** and at the other end to the distal end **200** of the pusher **180** so that actuation of the pusher **180** may selectively expand or retract the expandable assembly **220.** Typically, the catheter assembly **100** may be inserted into a delivery sheath (not shown in **FIG. 2**) so as to arrive at the desired location in heart **12.** Thereafter, the expandable assembly **220** may be expanded when protruding out of the delivery sheath by actuation of the pusher **180.**

The flexible circuit strip may have any suitable dimensions. For example, the length of the flexible circuit strip may be in the range of 10 mm to 60 mm, e.g., 30 mm, the width of the flexible circuit strip may be in the range of 0.25 mm to 3 mm, e.g., 0.72 mm, the thickness of the flexible circuit strips may be in the range of 0.005 mm to 0.14 mm.

In some examples, electrodes **260** extend to occupy a majority of the width of the flexible circuit strips by which they are hosted. Where, in some examples, electrode width is 0.25-3 mm, and/or electrode length is 0.25-3 mm.

The elongated resilient support elements may provide a shape of the expandable assembly **220** in the expanded form of the expandable assembly **220.** The elongated resilient support elements may include any suitable material, for example, but not limited to, Nitinol and/or shape-set Nitinol and/or Polyetherimide (PEI). As explained in further details herein, the present disclosure may provide that the respective elongated resilient support elements may extend in a hollow channel of the respective flexible circuit strips.

Electrodes **260** positioned on splines **240** of basket assembly **220** can be configured to determine the location of basket assembly **220** and/or to measure a physiological property such as local surface electrical potentials at respective locations on cardiac tissue. Additionally, or alternatively, the electrodes can also be used to deliver ablation energy RF and/or IRE to cardiac tissue. Examples of materials ideally suited for forming electrodes **220** include gold, platinum and palladium and their respective alloys.

In other examples, the catheter assembly may include a tubular shaft having a distal end, a coupler connected to the distal end of the tubular shaft. The catheter assembly may also include an expandable assembly **220** (also referred to generally as expandable end effector) comprising a plurality of flexible splines (only one label for the sake of simplicity). Each flexible spline may include a flexible circuit strip (PCB) including multiple electrodes disposed thereon and a corresponding elongated resilient support element extending along a given length of the flexible polymer circuit strip.

The flexible splines may be coupled at both ends to the coupler (also referred to as spline retention hub) and may be configured to expand radially into a basket shape when unconstrained. Typically, the catheter assembly may be inserted into a delivery sheath so as to arrive at the desired location in heart. Thereafter, the expandable assembly may expand when protruding out of the delivery sheath i.e. only by being advanced out of the delivery sheath. In other words, in these other examples, the expandable basket assembly may be configured to transition from the collapsed form to the expanded form solely by release of radial constraints on the flexible splines. The flexible splines may also be configured to retract when being pulled into the delivery sheath.

The catheter assembly may further include a contact force sensor to determine contact force of the splines against cardiac tissues. Details of the contact force sensor are shown and described in US Patent Application Publication No. US20210077180A1 published Mar. 18, 2021, which disclosure is incorporated by reference herein.

Reference is now made to **FIG. 3****,** which is a schematic view of flexible polymer circuit strips for use e.g. in the catheter assembly **100** of **FIG. 2****.** The flexible polymer circuit strips **245** may be formed from a polymer, such as polyimide. Circuit strips **245** may be connected to each other by polyimide, or assembled as individual pieces that are held in proper alignment and secured to coupler **160.** Respective first ends **420** of the respective flexible polymer circuit strips **245** include an electrical connection array **600.** An inset **62** shows that the electrical connection array **600** includes electrical contacts **640** thereon (only some labeled for the sake of simplicity). The electrical contacts **640** are connected via traces (not shown) on one side of top layer of the flexible polymer circuit strips **245** to respective ones of the electrodes **260** disposed on the other side of the top layer of the flexible polymer circuit strips. Away from the region of the first ends **420,** the flexible polymer circuit strips **245** are separate from each other to allow the flexible polymer circuit strips **245** to form the expandable assembly **220** (**FIG. 2**) when connected to the catheter assembly **100.** Wires (not shown) may connect the electrodes **260** to control circuitry (not shown) via the electrical contacts **640.** The wires may be disposed in lumens (not illustrated) of the elongated deflectable element.

In some examples, one or more pad dimensions are smaller than electrode dimensions. Where the surface area the pad presents for contact (e.g., by a measurement probe or joining tool) is smaller than that presented by the electrodes. For example, a pad upper surface area being 1-20% of that of the electrodes. For example, a pad width 194 being 0.05-0.5 mm, and/or a pad length 0.1-1 mm.

A surface area **620** of the flexible circuit strips on which pads **420** are disposed may be small and/or the density of pads on the area may be high. For example, the area having a width of 0.25-3 mm and a length of 0.5-6 mm and/or the proportion of the surface area occupied by pads is 20-50%.

As explained in more details below, the flexible polymer circuit strip is generally formed from multiple insulating layers including a top layer (also referred to as first layer), a middle layer (also referred to as second layer) and a bottom layer (also referred to as third layer). A conductive trace layer may be positioned between the middle insulating layer and the top layer of the flexible polymer circuit strip **245.** The conductive trace layer may include circuit traces which connect the electrodes **260** to the connection array **600.** The top layer includes the electrodes **260** and optionally one or more contact pads. The circuit traces on the middle layer may connect each electrode **260** to a corresponding electrical contact **640** using vias (not shown). The circuit traces for the electrodes **260** may be within the range of about 0.005 mm to 0.1 mm wide (e.g., 0.025 mm) with a spacing in the range of about 0.005 mm to 0.1 mm (e.g., 0.025 mm). The thickness of the traces may be in the range of about 0.005 mm to 0.100 mm (e.g., 0.010 mm). In some embodiments, the circuit traces may have the same width and spacing.

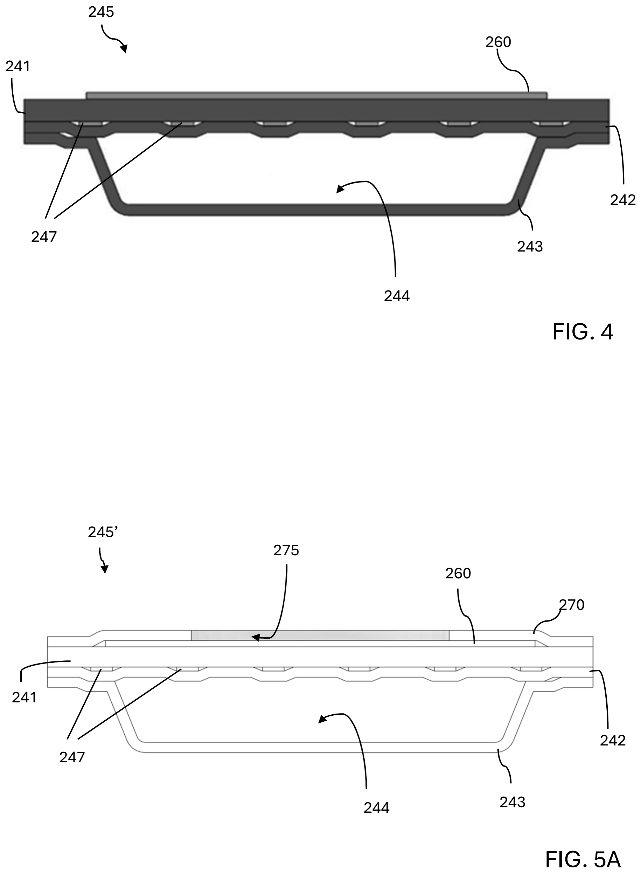

**FIG. 4** illustrates a cross section view of a flexible circuit strip **245** in accordance with examples of the present disclosure. Flexible circuit strip **245** may include a (top) first insulating layer **241,** a second (middle) insulating layer **242** and a third (bottom) insulating layer **243.** The first, second and third insulating layers **241, 242, 243** may be formed from a polymer, such as polyimide. The first, second and third insulating layers **241, 242, 243** may have elongated shapes so as to form a circuit strip. The first, second and third insulating layers **241, 242, 243** may have corresponding lateral and longitudinal dimensions so that they can be properly aligned and registered to each other e.g. in a lamination process. A thickness of the first insulating layer **241** may be larger than one of the second and third insulating layers **242, 243.**

A proximal end of the first insulating layer **241** may include an electrical connection array (also referred to as contact array) as described in relation to **FIG. 3****.** The electrical connection array may be positioned at a proximal end of the outer surface of the first insulating layer **241.** Electrodes **260** may be positioned along a length of an outer surface of the first insulating layer **241.** The electrodes **260** may be disposed away from the proximal end of the insulating layer **241.** The electrodes may be formed of a first conductive material such as gold.

The flexible circuit strip **245** may further include a conductive trace layer positioned between the first insulating layer **241** and the second insulating layer **242.** The conductive trace layer may include longitudinal circuit traces **247** for carrying electrical signals. The longitudinal circuit traces **247** may be configured to carry electrical signals between the electrodes **260** and the electrical connection array. The electrodes **260** may be connected to the circuit traces **247** using vias extending through the first and second insulating layers **241, 242.** The conductive circuit traces **247** may be formed of the same first conductive material as the electrodes. In some embodiments, the conductive circuit traces may be formed of a second conductive material different from the first conductive material.

The flexible circuit strip **245** may further include a longitudinal hollow channel **244** formed between the second and third insulating layers **242, 243** (or in some other examples between the first and second insulating layers). The longitudinal hollow channel **244** may be configured to receive an elongated support element (not shown) extending substantially along the full length of the flexible circuit strip. The elongated support element may be formed of resilient material and may include, for example, Nitinol and/or shape-set Nitinol and/or Polyetherimide (PEI). The elongated support element may be arranged in the longitudinal hollow channel to have play, allowing for longitudinal adjustment therein. The elongated support element may be adhered to the longitudinal hollow channel using epoxy so as to provide a limited adhesion allowing for residual longitudinal play. In some examples, the elongated support element may be adhered so as to be fixed within the longitudinal channel. The longitudinal hollow channel may have a constant cross section geometry. A cross sectional shape of the longitudinal hollow channel may be trapezoidal, rectangular, circular or elliptical. In some examples, the longitudinal hollow channel may have a variable cross section along the length of the flexible circuit strip and may be configured to accommodate an elongated support element with a varying diameter. In some examples, the longitudinal hollow channel may comprise reinforcement features to enhance structural integrity of the flexible circuit strip.

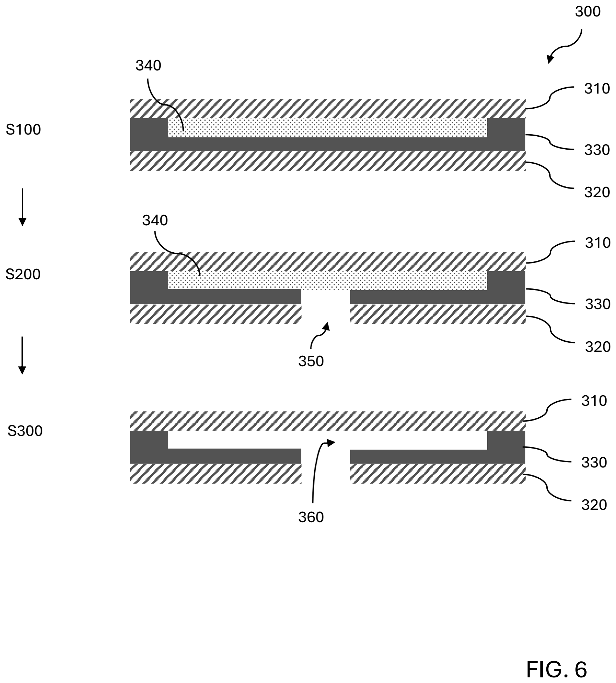

As explained in more details with reference to **FIG. 6****,** the longitudinal hollow channel **244** may be created by etching away a conductive channel layer clad to a surface of the second insulating layer facing the third insulating layer. The conductive channel layer may be formed of a third conductive material (e.g. copper) different from the first and/or second conductive material. This may allow to etch the conductive layer clad while preserving the electrodes and/or circuit traces.

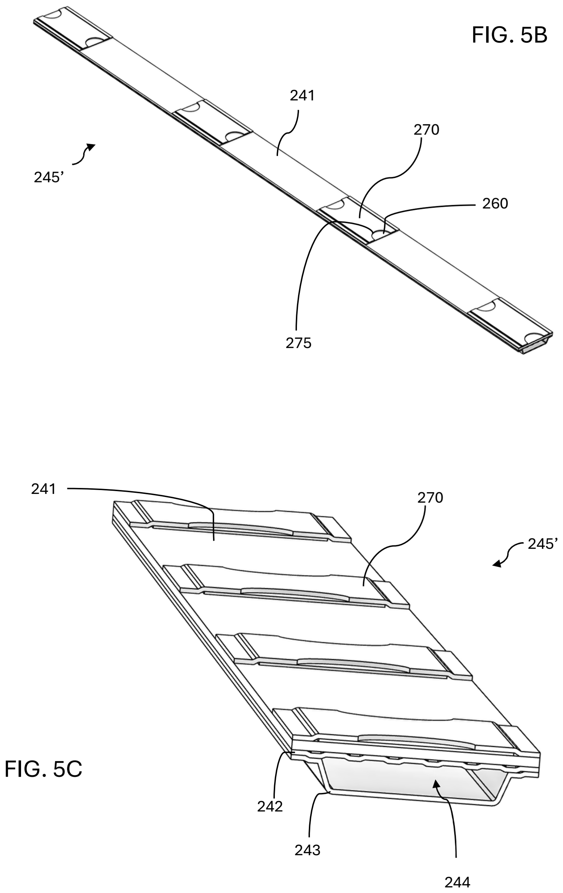

**FIGS. 5A-5C** illustrate views of a flexible circuit strip **245'** in accordance with examples of the present disclosure. In **FIGS. 5A-65,** the same reference numbers are used to represent elements that are similar to those in the previously described **FIG. 4****.** For the sake of brevity, the detailed description of these elements is not repeated here. Only the additions or differences are elaborated upon. As illustrated in **FIGS. 5A-5C****,** the flexible circuit strip **245'** additionally includes a plurality of insulating covers **270** respectively covering the plurality of electrodes **260.** The insulating covers **270** may include one or more apertures **275.** The insulating covers **270** may be positioned over the electrodes **260** so as to expose selected portions of the electrodes **260.** The one or more apertures **275** may have a semi-circular shape so as to expose a semi-circular portion of the electrode. In some examples, the selected portions of the electrodes may be positioned at lateral edges of the electrodes **260.**

The **flexible** circuit strips **245, 245'** described with reference to **FIGS.** 4 and **5A****-5C** may be suitable for integration into the end effector and catheter assemblies detailed above with reference to **FIG. 2****.** In particular, the flexible splines **240** described herein above may include the circuit strips **245, 245'** and an elongated support element may be disposed in the longitudinal hollow channel **244** of said circuit strips **245, 245'.**

**FIG. 6** **illustrates** steps of a method for fabricating a flexible circuit strip in accordance with examples of the present disclosure. In a first step **S100,** an elongated multilayer laminate **300** may be provided. The multilayer laminate **300** may comprise an insulating layer **310** formed of a flexible polymer such as polyimide, a conductive channel layer **340** clad onto the insulating layer **310** formed of a conductive material such as copper, an adhesive layer **330** formed of an adhesive such as epoxy and another insulating layer **320** formed of polymer such as polyimide. In a second step **S200,** the multilayer laminate **300** may be drilled to form an opening **350** through the other polymer layer **320** and the adhesive layer **330** so as to expose the conductive channel layer **340.** This step may be performed using precision drilling techniques such as laser drilling or mechanical drilling to ensure accuracy and consistency. In a third step **S300,** the conductive channel layer **340** may be etched so as to form a longitudinal hollow channel in the elongated multilayer laminate **300.** The etching process may involve chemical etching using an etchant solution that selectively removes the conductive material of the conductive channel (e.g. copper) while preserving other conductive materials optionally present on the laminate (e.g. gold traces and/or electrodes), or plasma etching for more precise control.

The method described herein may be used to form the longitudinal hollow channel in the flexible circuit strips as described herein above. The insulating layers **310, 320** may correspond to the middle insulating layer and bottom insulating layer as described previously. The resulting hollow channel may be configured to accommodate an elongated support element, such as a nitinol wire.

### EXAMPLES

Following is a non-exclusive list of some exemplary examples of the disclosure. The present disclosure also includes examples which include fewer than all the features in an example and examples using features from multiple examples, even if not listed below.

### Example 1

A flexible circuit strip (245) for use in a catheter assembly (100), comprising:
**(a)** A first insulating layer (241), a second insulating layer (242) and a third insulating layer (243), each having an elongated shape and bonded together such that the second insulating layer is between the first and third insulating layers;
**(b)** a conductive trace layer positioned between the first and second insulating layers, the conductive trace layer including longitudinal circuit traces (247) for carrying electrical signals;
**(c)** electrodes (260) positioned along a length of an outer surface of the first insulating layer and electrically connected to the circuit traces;
**(d)** a longitudinal hollow channel (244) formed between any two of the first, second and third layers (241, 242, 243), the longitudinal hollow channel (244) being configured to receive an elongated support element.

### Example 2

The flexible circuit strip (245) of Example 1, further comprising a contact array (600) positioned at a proximal end of the outer surface of the first insulating layer (241), wherein the electrodes (260) are connected to the contact array (600) using the circuit traces (247).

### Example 3

The flexible circuit strip (245) of Example 2, wherein the connection between the circuit traces (247) and the electrodes (260) is established through vias extending through the first insulating layer (241).

### Example 4

The flexible circuit strip (245) of any of the preceding Examples, wherein each electrode (260) is covered by an insulating cover (270) including one or more apertures (275), the insulating cover (270) being positioned over the electrode (260) to expose selected portions of the electrodes (260).

### Example 5

The flexible circuit strip (245) of any of the preceding Examples, wherein the electrodes (260) and the circuit traces (247) comprise gold.

### Example 6

The flexible circuit strip (245) of any of the preceding Examples, wherein the first, second, and third insulating layers (241, 242, 243) comprises polyimide.

### Example 7

The flexible circuit strip (245) of any of the preceding Examples, wherein the conductive channel layer comprises gold.

### Example 8

The flexible circuit strip (245) of any of the preceding Examples, wherein the first insulating layer (241) is thicker than the second and third insulating layers (242, 243).

### Example 9

An expandable end effector for use in a catheter assembly (100), the expandable end effector (220) comprising:
**(a)** a spline retention hub (160);
**(b)** an expandable basket assembly (220) coupled to the spline retention hub (160), the expandable basket assembly (220) comprising one or more flexible spline (240) coupled to the spline retention hub (160) and being configured to bow radially outward from a collapsed form to an expanded form, each flexible spline (240) including:
   i. a flexible circuit strip (245) according to any of the preceding Examples;
   ii. an elongated support element disposed in the longitudinal hollow channel (244).

### Example 10

The expandable end effector of Example 9, wherein the elongated support member is arranged to have play within the longitudinal hollow channel (244), allowing for longitudinal adjustment therein.

### Example 11

The expandable end effector of Example 9, wherein the elongated support member is fixed within the longitudinal hollow channel (244).

### Example 12

The expandable end effector of any of Examples 9 to 11, wherein the expandable basket assembly (220) comprises a first flexible spline having a proximal end coupled to the spline retention hub (160) and a distal end of a first flexible spline coupled to a distal end of a second flexible spline.

### Example 13

The expandable end effector of any of Example 9 to 11, further comprising a pusher (180) disposed coaxially within the spline retention hub (160) and configured to be translated relative thereto along a hub longitudinal axis; each of the one or more flexible spline (240) having a proximal end coupled to the spline retention hub (160) and a distal end coupled to a distal end of the pusher (180), the one or more flexible spline (240) being configured to bow radially outward when the pusher (180) is retracted relative to the spline retention hub (160), thereby expanding the expandable basket assembly (220) from a collapsed form to an expanded form.

### Example 14

The expandable end effector of any of Example 9 to 11, wherein the one or more flexible spline (240) is configured to bow radially outward upon release of radial constraint.

### Example 15

The expandable end effector of Example 14, wherein the one or more flexible spline (240) comprises a proximal end and a distal end both coupled to the spline retention hub (160).

### Example 16

The expandable end effector of any of Examples 9 to 15, comprising a plurality of flexible splines (240) forming a basket in the expanded form.

### Example_17

The expandable end effector of any of Examples 9 to 16, wherein the elongated support element is fitted to the longitudinal hollow channel (244) and secured loosely using an adhesive so as to have longitudinal play.

### Example 18

The expandable end effector of any of Examples 9 to 17, wherein the elongated support element comprises shape-set nitinol.

### Example 19

A catheter assembly (100) comprising:
- a tubular shaft (140) extending along a longitudinal axis extending from a proximal portion of the tubular shaft (140) to a distal portion of the tubular shaft (140); and
- an expandable end effector according to any of Examples 9 to 18.

### Example 20

A method of manufacturing a flexible circuit strip according to any of examples 1 to 8, the method comprising:
**(a)** providing (S100) an elongated multilayer laminate (300), wherein the multilayer laminate comprises an insulating layer (310) comprising a flexible polymer, a conductive channel layer (340) clad onto the insulating layer (310) and comprising a conductive material, an adhesive layer (330) and another insulating layer (320) comprising said flexible polymer;
**(b)** drilling (S200) the multilayer laminate 300) to form an opening (350) through said another polymer layer (320) and the adhesive layer (330) to expose the conductive channel layer (340);
**(c)** etching the conductive channel layer (340) to form a longitudinal hollow channel (360) in the elongated multilayer laminate (300).

Those skilled in the art to which the present disclosure pertains, can appreciate that while the present disclosure has been described in terms of preferred examples, the concept upon which this disclosure is based may readily be utilized as a basis for the designing of other structures, systems and processes for carrying out the several purposes of the present disclosure.

Also, it is to be understood that the phraseology and terminology employed herein are for the purpose of description and should not be regarded as limiting. It should be noted that the words "comprising", "including" and "having" as used throughout the appended claims are to be interpreted to mean "including but not limited to". The indefinite articles "a" and "an," as used herein in the specification and in the claims, unless clearly indicated to the contrary, should be understood to mean "at least one." The phrase "and/or," as used herein in the specification and in the claims, should be understood to mean "either or both" of the elements so conjoined, i.e., elements that are conjunctively present in some cases, and disjunctively present in other cases. The term "each" may not be exclusively understood as referring to each and every, and when technically relevant may also refer to "at least some".

All patents and patent applications mentioned in this specification are herein incorporated in their entirety by reference into the specification, to the same extent as if each individual patent or patent application was specifically and individually indicated to be incorporated herein by reference. In addition, citation or identification of any reference in this application shall not be construed as an admission that such reference is available as prior art to the present disclosure.

It is important, therefore, that the scope of the present disclosure is not construed as being limited by the illustrative examples set forth herein. Other variations are possible within the scope of the present disclosure as defined in the appended claims. Other combinations and sub-combinations of features, functions, elements and/or properties may be claimed through amendment of the present claims or presentation of new claims in this or a related application. Such amended or new claims, whether they are directed to different combinations or directed to the same combinations, whether different, broader, narrower or equal in scope to the original claims, are also regarded as included within the subject matter of the present description.

## Claims

1. A flexible circuit strip (245) for use in a catheter assembly (100), comprising:
(a) a first insulating layer (241), a second insulating layer (242) and a third insulating layer (243), each having an elongated shape and bonded together such that the second insulating layer is between the first and third insulating layers;
(b) a conductive trace layer positioned between the first and second insulating layers, the conductive trace layer including longitudinal circuit traces (247) for carrying electrical signals;
(c) electrodes (260) positioned along a length of an outer surface of the first insulating layer and electrically connected to the circuit traces;
(d) a longitudinal hollow channel (244) formed between any two of the first, second and third layers (241, 242, 243), the longitudinal hollow channel (244) being configured to receive an elongated support element.

2. The flexible circuit strip (245) of claim 1, further comprising a contact array (600) positioned at a proximal end of the outer surface of the first insulating layer (241), wherein the electrodes (260) are connected to the contact array (600) using the circuit traces (247), optionally wherein the connection between the circuit traces (247) and the electrodes (260) is established through vias extending through the first insulating layer (241).

3. The flexible circuit strip (245) of any preceding claim, wherein each electrode (260) is covered by an insulating cover (270) including one or more apertures (275), the insulating cover (270) being positioned over the electrode (260) to expose selected portions of the electrodes (260).

4. The flexible circuit strip (245) of any preceding claim, wherein the electrodes (260) and the circuit traces (247) comprise gold.

5. The flexible circuit (245) strip of any preceding claim, wherein the first, second, and third insulating layers (241, 242, 243) comprises polyimide.

6. The flexible circuit strip (245) of any preceding claim, wherein the conductive trace layer comprises gold.

7. The flexible circuit strip (245) of any preceding claim wherein the first insulating layer (241) is thicker than the second and third insulating layers (242, 243).

8. An expandable end effector for use in a catheter assembly (100), the expandable end effector (220) comprising:
(a) a spline retention hub (160);
(b) an expandable basket assembly (220) coupled to the spline retention hub (160), the expandable basket assembly (220) comprising one or more flexible splines (240) coupled to the spline retention hub (160) and being configured to bow radially outward from a collapsed form to an expanded form, each flexible spline (240) including:
iii. a flexible circuit strip (245) comprising:
- a first insulating layer (241), a second insulating layer (242) and a third insulating layer (243), each having an elongated shape and bonded together such that the second insulating layer is between the first and third insulating layers;
- a conductive trace layer positioned between the first and second insulating layers, the conductive trace layer including longitudinal circuit traces (247) for carrying electrical signals;
- electrodes (260) positioned along a length of an outer surface of the first insulating layer and electrically connected to the circuit traces;
- a longitudinal hollow channel (244) formed between any two of the first, second and third insulating layers (241, 242, 243), the longitudinal hollow channel (244) being configured to receive an elongated support element;
iv. an elongated support element disposed in the longitudinal hollow channel (244).

9. The expandable end effector of claim 8, wherein the elongated support element is (i) arranged to have play within the longitudinal hollow channel (244), allowing for longitudinal adjustment therein or (ii) fixed within the longitudinal hollow channel (244).

10. The expandable end effector of claim 8 or claim 9, wherein the expandable basket assembly (220) comprises a first flexible spline having a proximal end coupled to the spline retention hub (160) and a distal end of a first flexible spline coupled to a distal end of a second flexible spline.

11. The expandable end effector of claim 8 or claim 9, further comprising a pusher (180) disposed coaxially within the spline retention hub (160) and configured to be translated relative thereto along a hub longitudinal axis; each of the one or more flexible spline (240) having a proximal end coupled to the spline retention hub (160) and a distal end coupled to a distal end of the pusher (180), the one or more flexible spline (240) being configured to bow radially outward when the pusher (180) is retracted relative to the spline retention hub (160), thereby expanding the expandable basket assembly (220) from a collapsed form to an expanded form.

12. The expandable end effector of claim 8 or claim 9, wherein the one or more flexible spline (240) is configured to bow radially outward upon release of radial constraint, optionally wherein the one or more flexible spline (240) comprises a proximal end and a distal end both coupled to the spline retention hub (160).

13. The expandable end effector of claim 8 to 12, comprising a plurality of flexible splines (240) forming a basket in the expanded form.

14. The expandable end effector of claim 8 to 13, wherein the elongated support element is fitted to the longitudinal hollow channel (244) and secured loosely using an adhesive so as to have longitudinal play.

15. The expandable end effector of claim 8 to 14, wherein the elongated support element comprises shape-set nitinol.

16. A catheter assembly (100) comprising:
- a tubular shaft (140) extending along a longitudinal axis extending from a proximal portion of the tubular shaft (140) to a distal portion of the tubular shaft (140); and
- an expandable end effector comprising:
(a) a spline retention hub (160) coupled to the distal portion of the tubular shaft;
(b) an expandable basket assembly (220) coupled to the spline retention hub (160), the expandable basket assembly (220) comprising one or more flexible spline (240) coupled to the spline retention hub (160) and being configured to bow radially outward from a collapsed form to an expanded form, each flexible spline (240) including:
i. a flexible circuit strip (245) comprising:
- a first insulating layer (241), a second insulating layer (242) and a third insulating layer (243), each having an elongated shape and bonded together such that the second insulating layer is between the first and third insulating layers;
- a conductive trace layer positioned between the first and second insulating layers, the conductive trace layer including longitudinal circuit traces (247) for carrying electrical signals;
- electrodes (260) positioned along a length of an outer surface of the first insulating layer and electrically connected to the circuit traces;
- a longitudinal hollow channel (244) formed between any two of the first, second and third insulating layers (241, 242, 243), the longitudinal hollow channel (244) being configured to receive an elongated support element;
ii. an elongated support element disposed in the longitudinal hollow channel.

17. A method of manufacturing a flexible circuit strip, comprising:
(a) providing an elongated multilayer laminate (300), wherein the multilayer laminate comprises an insulating layer (310) comprising a flexible polymer, a conductive channel layer (340) clad onto the insulating layer (310) and comprising a conductive material, an adhesive layer (330) and another insulating layer (320) comprising said flexible polymer;
(b) drilling the multilayer laminate (300) to form an opening (350) through said another polymer layer (320) and the adhesive layer (330) to expose the conductive channel layer (340);
(c) etching the conductive channel layer (340) to form a longitudinal hollow channel (360) in the elongated multilayer laminate (300).
